# EUROPÄISCHE PATENTSCHRIFT

(11) **EP 1 554 734 B1**
(45) Veröffentlichungstag und Bekanntmachung des Hinweises auf die Patenterteilung: **25.03.2009**
(21) Anmeldenummer: 02782706.2
(22) Anmeldetag: 09.10.2002
(51) Int. Cl.: H01F 1/00, A61K 51/12

(54) **STABILISIERTE SUPERPARAMAGNETISCHE TEILCHEN**
STABILIZED SUPERPARAMAGNETIC PARTICLES
PARTICULES SUPRAPARAMAGNETIQUES STABILISEES

(43) Veröffentlichungstag der Anmeldung: 20.07.2005
(73) Patentinhaber: Pilgrimm, Helga, 14193 Berlin (DE)
(72) Erfinder: PILGRIMM, Herbert, 14169 Berlin (DE)
(74) Vertreter: Walter, Wolf-Jürgen
(86) Internationale Anmeldenummer: PCT/DE2002/003862
(87) Internationale Veröffentlichungsnummer: WO 2004/034411

(56) Entgegenhaltungen:
- EP-B- 0 888 545
- DE-A- 4 427 821
- US-A- 5 928 958

## Beschreibung

Die Erfindung betrifft superparamagnetische Teilchen, die aus superparamagnetischen Eindomänenteilchen und Aggregaten von superparamagnetischen Eindomänenteilchen aus Eisenoxiden, Eisenmischoxiden oder Eisen bestehen, die auf ihrer Oberfläche stabilisiert sind und die in der Medizin oder medizinischen Diagnostik eingesetzt werden können.

In der EP 0772776 B1 werden superparamagnetische Teilchen beschrieben, die aus superparamagnetischen Eindomänenteilchen und Aggregaten von superparamagnetischen Eindomänenteilchen bestehen und die auf ihrer Oberfläche organische Substanzen gebunden haben, die gegebenenfalls weitere Bindungsstellen zur Kopplung von gewebespezifischen Bindungssubstanzen, diagnostischen oder pharmakologisch wirksamen Substanzen besitzen. Die superparamagnetische Teilchen setzen sich aus einem Gemisch von kleinen superparamagnetischen Eindomänenteilchen mit einer Teilchengröße im Bereich zwischen 3 und 50 Nanometer und stabilen, abbaubaren Aggregate aus kleinen superparamagnetischen Eindomänenteilchen mit einer Teilchengröße im Bereich zwischen 10 und 1000 Nanometer zusammen und bestehen aus Eisenhydroxid, Eisenoxidhydrat, Eisenoxid-, Eisenmischoxid- oder Eisen, die auf ihrer Oberfläche mono- und/oder polyhydroxylgruppenhaltige aromatische Substanzen, Polyglycerine, aminosäurenhaltige Substanzen, silikatgruppenhaltige Substanzen der Orthokieselsäure und deren Kondensationsprodukte und phosphatgruppenhaltigen Substanzen der Ortho- oder Metaphosphorsäure und deren Kondensationsprodukte gebunden tragen, die weitere Bindungsstellen aufweisen können.

In der EP 0888545 B1 werden superparamagnetische Eindomänenteilchen mit vergrößerter R1-Relaxivität und mit Oberflächen-Stabilisatorsubstanzen beschrieben, deren Teilchen aus Eisenhydroxid, Eisenoxidhydrat, Eisenoxid, Eisenmischoxid oder Eisen bestehen, eine Teilchengröße im Bereich zwischen 1 und 10 Nanometer, mit einem mittleren Teilchendurchmesser d₅₀ von 2 bis 4 Nanometer, besitzen und eine vergrößerte R₁-Relaxivität im Bereich von 2 bis 50 und ein Verhältnis der Relaxivitäten R₂/R₁ kleiner 5 haben. Auf ihrer Oberfläche sind niedermolekulare Stabilisatorsubstanzen, wie Citronensäure gebunden, die eine Aggregation und Sedimentation im Schwerefeld oder in einem Magnetfeld verhindern.

Der Erfindung liegt die Aufgabe zugrunde, den Bereich der Substanzen, die an der Oberfläche der Eindomänenteilchen gebunden sein können, zu erweitern, um die physikalisch, chemischen und physiologischen Eigenschaften der entstehenden Magnetteilchen den jeweiligen Anwendungsgebieten optimal anpassen zu können, wobei diese Substanzen stabil und leicht herstellbar sein sollen.

Die in der EP 0772776 B1 beschriebenen superparamagnetische Teilchen, die aus superparamagnetischen Eindomänenteilchen die auf ihrer Oberfläche organische Substanzen gebunden haben, lassen sich auch mit den in der EP 0888545 B1 beschriebenen niedermolekularen aliphatischen Di- und Polycarbonsäuren, wie Äpfelsäure, Weinsäure, Zitronensäure, Asparaginsäure, gegen Sedimentation im Schwerefeld der Erde oder einem Magnetfeld stabilisierten. Auch die in der EP 0772776 B1 beschriebenen Aggregate von superparamagnetischen Eindomänenteilchen lassen sich ebenfalls gegen eine Sedimentation im Schwerefeld der Erde, z.B. durch die in der EP 0888545 B1 beschriebene niedermolekulare Citronensäure stabilisieren.

Es wurde gefunden, dass stabilisierte superparamagnetische Teilchen, bestehend aus superparamagnetischen Eindomänenteilchen aus Eisenhydroxid, Eisenoxidhydrat, Eisenoxid-Eisenmischoxid- oder Eisen, die eine Teilchengröße im Bereich von 2 und 50 Nanometer haben,
oder Aggregaten davon, die eine Teilchengröße im Bereich von10 bis 1000 Nanometer haben, oder Gemischen davon,
die jeweils stabilisiert sind auf ihrer Oberfläche durch aliphatische Di- oder Polycarbonsäuren oder Derivate davon,
auf ihrer Oberfläche geladene Ionen chemischer Elemente gebunden tragen können, wobei die Ionen bis zu einem Metallionengehalt von 5 Mol% des Eisengehaltes der Eindomänenteilchen gebunden sind und positiv geladene Metallionen sind, die aus der Gruppe ausgewählt sind, die aus Ionen der chemischen Elemente Kupfer, Silber, Gold, Eisen, Nickel, Kobalt, Gallium, Thallium, Bismut, Palladium, Rhenium, Rhodium, Ruthenium, Platin, Technetium, Indium, Iridium, Osmium, Radium, Selen, Vanadium,
Yttrium, Zirkon, seltenen Erden, Gemischen davon und radioaktiven Isotopen dieser Elemente besteht. Die Ionen gehen mit der Oberfläche der superparamagnetischen Teilchen sehr stabile Bindungen ein, die die Sedimentationsstabilität der superparamagnetischen Eindomänenteilchen und Aggregate in bestimmten Konzentrationsbereichen nicht beeinflussen.

Die Stabilitätseigenschaften der metallionenhaltigen Dispersionen wurden bis zu einem Gehalt an Metallionen von bis zu 10 Mol-% des Eisengehaltes der Magnetteilchen untersucht.

Dabei wurde bei allen untersuchten Kationenarten bis zu einem Metallionengehalt von 5 Mol-% des Eisengehaltes der Magnetteilchen die Stabilität der Dispersionen nicht verändert. Die mit der Atom-Absorptions-Spektrokopie (AAS) gemessenen Ionenkonzentrationen der zugesetzten Metallionen im Ultrafiltrat der Dispersionen lagen überraschend bei allen. Proben unter der jeweiligen Nachweisgrenze der Meßmethode. Erst oberhalb von einem Metallionengehalt von 5 Mol-% des Eisengehaltes der Magnetteilchen verringert sich die Stabilität der Dispersionen in Abhängigkeit von der Elementart und dem Gehalt der zugesetzten Metallionen und die gemessene Ionenkonzentration im Ultrafiltrat der Dispersionen lag im Meßbereich der AAS.

In einer Ausführungsform der Erfindung sind die Metallionen aus der Gruppe der radioaktiven Isotope, bestehend aus ⁵²Fe, ⁶⁷Ga, ^{99m}Tc, ¹¹³In, ¹⁸⁸Rh, ¹⁹²Ir, ¹⁹⁸Au, ²⁰¹Tl und ²²³Ra ausgewählt.

Eine bevorzugte Gruppe von positiv geladenen Metallionen sind aus der Gruppe ausgewählt, die aus Metallionen der chemischen Elemente Kupfer, Silber, Gold, Platin, Palladium, Osmium, Rhenium, Rhodium, Ruthenium, Vanadium und Gemischen davon besteht.

Gegenstand der Erfindung sind auch stabilisierte superparamagnetische Teilchen, bestehend aus superparamagnetischen Eindomänenteilchen aus Eisenhydroxid, Eisenoxidhydrat, Eisenoxid-, Eisenmischoxid- oder Eisen, die eine Teilchengröße im Bereich von 2 und 50 Nanometer haben, oder Aggregaten davon, die eine Teilchengröße im Bereich von10 bis 1000 Nanometer haben, oder Gemischen davon, jeweils stabilisiert auf ihrer Oberfläche durch aliphatische Di- oder Polycarbonsäuren oder Derivate davon, die eine Aggregation und Sedimentation im Schwerefeld verhindern, **dadurch gekennzeichnet, dass** die superparamagnetischen Eindomänenteilchen auf ihrer Oberfläche geladene Ionen chemischer Elemente gebunden tragen, wobei die geladenen Ionen Nichtmetallionen sind, die über eine Polyethylenimin-Brücke an die Oberfläche der superparamagnetischen Eindomänenteilchen gebunden sind, vorzugsweise werden die radioaktiven Isotope ¹³N, ¹⁵O, ¹⁸F, ¹²³J oder Gemische davon auf diese Weise an die stabilisierten superparamagnetischen Teilchen gebunden.

Neben den geladenen Ionen chemischer Elemente können als weitere vorteilhafte Ausführungsform der Erfindung gegebenenfalls noch gewebespezifische Bindungssubstanzen auf den Oberflächen der superparamagnetischen Teilchen gebunden sein. Diese Substanzen können aus der Gruppe ausgewählt sein, bestehend aus Antigene, Antikörper, Ribonucleinsäuren, Desoxyribonucleinsäuren, Ribonucleinsäuresequenzen, Desoxyribonucleinsäure-sequenzen, Haptene, Avidin, Streptavidin, Protein A, Protein G, Endotoxin-bindende Proteine, Lectine, Selectine, Oberflächenproteine von Organellen, Viren, Mikroben, Algen, Pilze;

Neben den geladenen Ionen chemischer Elemente können als weitere vorteilhafte Ausführungsform der Erfindung gegebenenfalls noch pharmakologisch wirksamen Substanzen auf den Oberflächen der superparamagnetischen Teilchen gebunden sein, die aus der Gruppe ausgewählt sind, die Antitumorproteine, Enzyme, Antitumorenzyme, Antibiotika, Pflanzenalkaloide, Alkylierungsreagenzien, Antimetaboliten, Hormone und Hormonantagonisten, Interleukine, Interferone, Wachstumsfaktoren, Tumomekrosefaktoren, Endotoxine, Lymphotoxine, Urokinase, Streptokinase, Plasminogen-Streptokinase-Aktivator-Komplex, Gewebe- Plasminogen- Aktivatoren, Desmodus-Plasminogen-Aktivatoren, Makrophagen-Aktivierungs-Körper, Antisera, Blut und Zellbestandteile und deren Abbauprodukte und Derivate, Zellwandbestandteile von Organellen, Viren, Mikroben, Algen, Pilze und deren Abbauprodukte und Derivate, Proteaseninhibitoren, Alkylphosphocholine, radioaktive Isotope enthaltende Substanzen, Tenside, kardiovaskulare Pharmazeutika, Chemotherapeutika, gastrointestinale Pharmazeutika und Neuropharmazeutika umfasst.

Unter "Derivate von aliphatischen Di- oder Polycarbonsäuren" werden insbesondere monofunktionelle Ester bei Dicarbonsäuren oder mono- oder difunktionelle Ester bei Polycarbonsäuren verstanden, die C₁-C₁₈-Alkyteile, vorzugsweise C₁-C₄-Alkylteile enthalten.

Die Herstellung der superparamagnetischen Teilchen erfolgt in bekannter Weise durch eine Fällung aus einer Eisensalzlösung mit z.B. Ammoniakwasser und einer nachfolgenden gezielten Agglomeration der entstandenen superparamagnetischen Eindomänenteilchen. Dabei werden die superparamagnetischen Eindomänenteilchen in Wasser verrührt und bei einem pH-Wert von 1 bis 7 durch Erhitzen auf 80 bis 120°C, bei Temperaturen über 100°C im Autoklaven, zur Aggregation gebracht. Nach dem Abkühlen der Dispersion werden die Teilchen so lange gewaschen, bis die elektrische Leitfähigkeit des Filtrates kleiner als 10 µS/cm beträgt. Die so hergestellten superparamagnetischen Teilchen bilden sofort einen schnell sedimentierenden Niederschlag, der sich auch durch starkes Rühren oder durch Ultraschallbehandlung nicht in eine stabile Dispersion überführen läßt. Erst die Bindung von Stabilisatorsubstanzen auf der Oberfläche der superparamagnetischen Teilchen sorgt für eine Dispergierbarkeit Bei Citronensäure als Stabilisatorsubstanz reicht Rühren mit dem Glasstab, bei anderen Stabilisatorsubstanzen benötigt man einen stärkeren Energieeintrag, wie z.B. Erwärmen oder Einwirkung von Ultraschall, um stabile Dispersionen zu erhalten. Nach der Stabilisierung der superparamagnetischen Teilchen mit einer aliphatischen Di- oder Polycarbonsäure, z.B. mit Citronensäure, wird die Dispersion mit Basen, wie Natronlauge, Methylglucamin, auf einen pH-Wert von 7,0 eingestellt und gegen Wasser oder physiologische Kochsalzlösung dialysiert, um den überschüssigen Anteil an Elektrolyt zu entfernen.

Erfindungsgemäß erfolgt nun die Mischung der Dispersion der superparamagnetischen Teilchen, die einen Eisengehalt im Bereich von 0,001 Mol Fe/l bis 10 Mol Fe/l besitzen kann, und die in Wasser oder einem niedrigsiedenden organischen polaren Lösungsmittel dispergiert sein kann, mit einer wäßrigen Lösung von Ionen chemischer Elemente. Der anzuwendende Konzentrationsbereich der Lösungen der Ionen chemischer Elemente liegt im Bereich von 0,001 mmolar bis 1 molar. Das Mengenverhältnis von Ionen chemischer Elemente zu Eisen soll in der Mischung 10 Mol-% nicht überschreiten.

Es ist vorteilhaft, verdünnte Lösungen einzusetzen, z.B. zwischen 0,001 und 0,1 molare Lösungen, und diese langsam zuzusetzen, z.B. tropfenweise, um einen örtlichen großen Konzentrationsgradienten zu vermeiden.

Die Ionen chemischer Elemente, wie die positiv geladenen Metallionen der chemischen Elemente Kupfer, Silber, Gold, Eisen, Nickel, Kobalt, Gallium, Thallium, Bismut, Palladium, Rhenium, Rhodium, Ruthenium, Platin, Technetium, Indium, Iridium, Osmium, Radium, Selen, Vanadium, Yttrium, Zirkon, und seltene Erden, sowie Gemische davon, oder auch radioaktive Isotope dieser Metallionen, wie ⁵²Fe, ⁶⁷Ga, ^{99m}Tc, ¹¹³In, ¹⁸⁸Rh, ¹⁹²Ir, ¹⁹⁸Au, ²⁰¹Tl oder ²²³Ra, werden vor der Mischung mit den superparamagnetischen Teilchen in Wasser oder einem niedrigsiedenden organischen polaren Lösungsmittel, vorzugsweise in Wasser gelöst.

Die negativ geladenen Ionen chemischer Elemente, wie die radioaktiven Isotope ¹³N, ¹⁵O, ¹⁸F, ¹²³J, werden vor der Mischung mit den superparamagnetischen Teilchen in einer wäßrigen Polyethylenimin-Lösung gelöst. Der anzuwendende Konzentrationsbereich der Polyethylenimin-Lösung liegt im Bereich von 0,001 bis 1 molar und der anzuwendende Konzentrationsbereich der Lösungen der negativ geladenen Ionen chemischer Elemente liegt im Bereich von 0,001 mmolar bis 1 mmolar.

Die Mischung der geladenen Ionen chemischer Elemente mit den superparamagnetischen Teilchen erfolgt unter Rühren, wobei es wichtig ist, daß die wäßrige Dispersion der superparamagnetischen Teilchen vorgelegt und die wäßrige Lösung von Ionen chemischer Elemente allmählich, z.B. tropfenweise zugegeben wird. Die Mischung erfolgt in einem Temperaturbereich von 5°C bis 70 °C, bevorzugt bei Raumtemperatur, d.h. bei 20-25 °C.

Die stabilisierte superparamagnetische Teilchendispersion enthält nicht oder nur schwach aggregierte superparamagnetische Eindomänenteilchen. Diese bilden eine stabile magnetische Flüssigkeit, die sich leicht von den größeren superparamagnetischen Aggregaten durch deren Sedimentation in einem Magnetfeld entsprechender Stärke und Inhomogenität abtrennen läßt.

In einer einfachen Ausführung der magnetischen Separation stellt man ein Becherglas mit der magnetischen Dispersion auf einen Permanentmagneten mit einer magnetischen Flußdichte von 0,1 T und gießt nach einer Sedimentationszeit von ca. 30 min die überstehende magnetische Flüssigkeit ab. In dem Sediment zurück bleiben die superparamagnetischen Aggregate, die, je nach Teilchengröße, sich wieder spontan in der Dispersion verteilen oder als Bodensatz im Becherglas zurückbleiben. Bis zu Teilchengrößen von ungefähr 500 nm verteilen sich die superparamagnetischen Aggregate wieder spontan oder unter leichtem Rühren im wäßrigen Dispersionsmittel.

Für das erfindungsgemäße Verfahren wurde gefunden, daß Polyethylenimine auf der z.B. mit Citronensäure stabilisierten Oberfläche der superparamagnetischen Teilchen stabile Bindungen eingehen, die die Sedimentationsstabilität der superparamagnetischen Eindomänenteilchen und superparamagnetischen Aggregate in bestimmten Konzentrationsbereichen nicht beeinflussen. Mit diesen Polyethylenimin beschichteten Magnetteilchen lassen sich auch radioaktive Nichtmetallionen auf der Oberfläche der superparamagnetischen Teilchen binden. An die freien Amingruppen der Polyaminverbindungen können dann die o.g. kurzlebigen Radiopharmaka, wie ¹³N, ¹⁵O, ¹⁸F, ¹²³J, gebunden werden.

Ebenso wurde gefunden, daß Polyethylenimin auf den z.B. mit Citronensäure stabilisierten Oberflächen der superparamagnetischen Teilchen auch dann stabile Bindungen eingehen, die die Sedimentationsstabilität der superparamagnetischen Eindomänenteilchen und superparamagnetischen Aggregate in bestimmten Konzentrationsbereichen nicht beeinflussen, wenn die Polyethylenimine vorher mit den kurzlebigen Radiopharmaka, wie ¹³N, ¹⁵O, ¹⁸F, ¹²³J, gemischt und erst dann auf der Oberflächen der superparamagnetischen Teilchen gebunden werden.

Die stabilisierten superparamagnetischen Teilchen können zur Herstellung eines Bakteriostaticums oder Radiopharmakons verwendet werden, zur Herstellung eines Mittels zur Tumorschädigung, zur Herstellung eines Mittels zur Verhinderung von Restenosen, zur Herstellung eines Mittels zur Bekämpfung von Entzündungskrankheiten, zur Herstellung eines Mittels zur Funktionskontrolle von

Organen oder zur Herstellung eines Mittels zum magnetischen drug targeting oder zur Herstellung eines MR-Kontrastmittels. Die erfindungsgemäßen Teilchen sind auch als magnetische Ionenaustauscher und magnetische Adsorbentien für Separationsverfahren oder als Magnetteilchen für die in vitro Diagnostik verwendbar, gegebenenfalls unter Einwirkung von Magnetfeldern.

Die erfindungsgemäßen ionenhaltigen, vorzugsweise metallionenhaltigen superparamagnetische Teilchen können z. B. zur Herstellung eines Bakteriostatikums verwendet werden. So wirken superparamagnetische Teilchen, auf deren Oberfläche Silberionen gebunden wurden, stark bakterizid. Silberhaltige Eindomänenteilchen oder deren Aggregate können somit zur Herstellung eines Therapeutikums z. B. zur Behandlung von entzündlichen Erkrankungen des Magen-Darm-Traktes eingesetzt werden. Die silberhaltigen superparamagnetischen Teilchen werden am bakteriellen Entzündungsherd adsorbiert, die bakterielle Sauerstoffversorgung wird durch die Wirkung der geringen Silberionenkonzentration unterbunden, die Bakterien werden abgetötet.

Silberhaltige superparamagnetische Eindomänenteilchen und Aggregate, wie nach Beispiel 3 können, wie Untersuchungen an Ratten gezeigt haben, zur Herstellung eines oralen Therapeutikums zur Behandlung entzündlicher Magen-Darm-Erkrankungen Anwendung finden, wie Erkrankungen durch die Bakterienart Helicobacter pylori.

Sehr kleine silberhaltige superparamagnetische Eindomänenteilchen, wie nach Beispiel 4 können, wie Untersuchungen an Ratten gezeigt haben, auch zur Herstellung eines parenteralen Therapeutikums zur Behandlung bakterieller Entzündungsprozesse im Körper Anwendung finden. Die Toxizität der Probe war mit einer LD 50 von 3 mmol Eisen/kg Körpergewicht für therapeutische Anwendungen geeignet. Bei Verringerung der Silberionenkonzentration ist mit einer Verringerung der Toxizität zu rechnen.

Ein Vorteil dieser stark bakteriziden, silberhaltigen Eindomänenteilchen oder deren Aggregate ist, daß mit Hilfe der Kernspin-Tomographie der Adsorptionsort und die adsorbierte Menge der Magnetteilchen diagnostizierbar sind.

Radioaktive superparamagnetischen Teilchen können zur Herstellung eines parenteralen Radiopharmakons dienen, das sowohl zur Diagnose und Therapie von vulnerablen Plaques als auch zur Diagnose und Therapie der Restenose nach Ballondilatation oder Stentimplantation anwendbar ist. Durch den T1- und T2-Effekt der sehr kleinen superparamagnetischen Eindomänenteilchen im Sinne der EP 0888545 B1 (vergrößerte R₁-Relaxivität im Bereich von 2 bis 50 und ein Verhältnis der Relaxivitäten R₂/R₁ kleiner 5), der hier ebenfalls zu verzeichnen ist, ist die Untersuchung der Anreicherung der Teilchen in den Gefäßwänden mit Hilfe der Kernspin-Tomographie möglich. Die therapeutische Wirkung der radioaktiven superparamagnetischen Teilchen zur Diagnose und Therapie von vulnerablen Plaques und zur Verhinderung von Restenosen nach Ballondilatation oder Stentimplantation liegt in der Zerstörung der für das wieder wachsen verantwortliche Zellen in den Plaques an den Gefäßwänden. So wird das parenterale Radiopharmakon nach der Entfernung der Plaques und nach der Ballondilatation oder Stentimplantation direkt durch eine Kanüle in den untersuchten Gefäßbereich gespritzt, um so, durch die Zerstörung der für die Plaquebildung verantwortlichen Gefäßzellen, die Restenose zu verhindern.

Radioaktive superparamagnetischen Teilchen mit gewebespezifischen Antikörpern können als Radiopharmakon zur Bekämpfung spezifischer Tumorarten eingesetzt werden, da nach parenteraler Injektion der Teilchen die gewebespezifischen Antikörper an den entsprechenden Rezeptoren der Tumorzellen andocken und die radioaktiven Bestandteile der Magnetteilchen die Tumorzellen zerstören.

Eine Diagnose und Therapie von Glioblastomen mit radioaktiv citratbeschichteten kleinen superparamagnetischen Eindomänenteilchen wird dadurch möglich.

Die superparamagnetischen Teilchen können auch zur in vitro Diagnostik oder als magnetische Ionenaustauscher und magnetische Adsorbentien zur Abtrennung von Ionen, organischen Molekülen, Makromolekülen, Zellen, Viren u.s.w. in der Biotechnologie, Abwasserreinigung oder sonstigen Stofftrennungsverfahren verwendet werden, wenn auf der Oberfläche der Teilchen die entsprechenden Ionenaustauschergruppen und Adsorbentien gebunden werden. Metallionenhaltige superparamagnetische Teilchen können auch zur Herstellung von extrem kleinen Metallteilchen Verwendung finden, indem die Eisenoxidteilchen in Gegenwart reduzierend wirkender Substanzen durch verdünnte Säuren aufgelöst werden. Die Herstellung von Katalysatoren mit großen Oberflächen ist ebenfalls möglich.

An Beispielen sollen Herstellung und Eigenschaften der erfindungsgemäßen superparamagnetischen Teilchen erläutert werden. In den Beispielen 1 und 2 werden die Muster 1 und 2 hergestellt, auf die nachfolgend Metallionen aufgebracht werden.

### Muster 1 (Beispiel 1):

Eisen (III)-chlorid (270 g) und Eisen(II)-sulfat (153 g) werden in 1 I dest. Wasser gelöst. Durch Zugabe von Natronlauge wird unter Rühren ein pH-Wert von 9,5 eingestellt. Nach erfolgter Fällung wird die Dispersion unter Rühren mit Salzsäure auf den pH-Wert von 5,0 eingestellt und auf 100°C erwärmt. Nach dem Abkühlen der Dispersion wird der Niederschlag gewaschen, bis das Filtrat eine elektrische Leitfähigkeit von < 10 µS/cm besitzt. Die Stabilisierung der superparamagnetischen Teilchen erfolgt durch Mischen der Teilchen mit einer wäßrigen Lösung von 120 g Citronensäure bei Raumtemperatur. Die Dispersion wird durch Zugabe von Natronlauge auf einen pH-Wert von 7,0 eingestellt und die nicht gebundenen Salze mit dest. Wasser dialysiert, bis das Dialysat eine elektrische Leitfähigkeit von < 10 µS/cm besitzt. Zur Entfernung größerer oder schwach aggregierter superparamagnetischer Teilchen wird die Dispersion 10 min bei 10.000 U/min zentrifugiert und das Zentrifugat durch Ultrafiltration mit einem 40kD-Filter auf einen Eisengehalt von ca. 2 Mol/l aufkonzentriert.

Die superparamagnetischen Eindomänenteilchen haben einen mittleren Teilchendurchmesser von ca. 16 nm. Im Bodensatz der Zentrifuge befinden sich die superparamagnetischen Teilchenaggregate, die einen mittleren Teilchendurchmesser von ca. 100 nm haben.

Typische Analysendaten der sehr kleinen superparamagnetischen Eindomänenteilchen sind:

| | |
|---|---|
| Teilchendurchmesser d50 | 8 nm |
| Gesamtdurchmesser | |
| mit Stabilisator: | 16 nm |
| Eisen(II)-Gehalt | 16 % |
| T1-Relaxivität | 12 l/mmol s |
| T2-Relaxivität | 25 l/mmol s |
| Verhältnis der Relaxivitäten R2/R1 | 2,05 |

### Muster 2 (Beispiel 2):

Eisen(III)-chlorid (270 g) und Eisen(II)-chlorid(119 g) werden in 1 I dest. Wasser gelöst. Durch Zugabe von Ammoniakwasser wird unter Rühren der pH-Wert der Lösung auf 9,6 eingestellt. Nach erfolgter Fällung wird die Dispersion 10 Minuten gerührt, mit einer Lösung von 120 g Citronensäure in 500 ml Wasser versetzt und 10 min gerührt. Nach dem Abkühlen der Dispersion wird der Niederschlag gewaschen, bis das Filtrat eine elektrische Leitfähigkeit von < 10 µS/cm besitzt. Der Feststoff wird in 300 ml Wasser Verrührt und 10 min mit Ultraschall von 100 W Leistung dispergiert. Die entstehende Dispersion wird 30 min auf einen Permanentmagneten mit einer magnetischen Flußdichte von 0,1 T sedimentiert und der Überstand von magnetischer Flüssigkeit abgegossen. Der Überstand enthält überwiegend stabilisierte superparamagnetische Eindomänenteilchen. Das Sediment auf dem Permanentmagneten enthält die superparamagnetischen abbaubaren Aggregate. Die Dispersion auf einen pH-Wert von 7,0 eingestellt und die nicht gebundenen Salze mit einer physiologischen Kochsalz-Iösung, bis das Dialysat einen Ammoniumionengehalt von <0,001g/l besitzt. Zur Entfernung größerer oder schwach aggregierter superparamagnetischer Teilchen wird die Dispersion 10 min bei 10.000 U/min zentrifugiert und das Zentrifugat durch Ultrafiltration mit einem 40kD-Filter auf einen Eisengehalt von ca. 2 Mol/l aufkonzentriert.

Die superparamagnetischen Eindomänenteilchen haben einen mittleren Teilchendurchmesser von ca. 14 nm. Im Bodensatz der Zentrifuge befinden sich die superparamagnetischen Teilchenaggregate, die einen mittleren Teilchendurchmesser von ca. 80 nm haben.

Typische Analysendaten der sehr kleinen superparamagnetischen Eindomänenteilchen sind:

| | |
|---|---|
| Teilchendurchmesser d50 | 4 nm |
| Gesamtdurchmesser | |
| mit Stabilisator: | 8 nm |
| Eisen(II)-Gehalt | 14 % |
| T1-Retaxivität | 19 l/mmol s |
| T2-Relaxivität | 36 l/mmol s |
| Verhältnis der Relaxivitäten R2/R1 | 1,89 |

### Beispiel 3:

Zu 20 ml der superparamagnetischen Aggregate von Beispiel 1, mit einem Eisengehalt von 2 Mol/l, werden tropfenweise 2 ml einer 0,1 molaren Silbernitrat-Lösung unter Rühren bei 25 °C beigemischt. Die überschüssige Elektrolytlösung wird durch Dialyse mit einem 40kD-Filter mit dest. Wasser dialysiert, bis das Dialysat eine elektrische Leitfähigkeit von < 10 µS/cm besitzt. Die entstehende Dispersion ist sedimentationsstabil und kann nach entsprechender pharmazeutischer Formulierung als Bakteriostatikum bei bakteriellen Erkrankungen des Magen-Darm-Traktes verwendet werden. Die Adsorption der superparamagnetischen Aggregate im Magen-Darm-Trakt kann mit Hilfe der Kernspin-Tomographie beobachtet werden.

### Beispiel 4:

Zu 20 ml der kleinen superparamagnetischen Eindomänenteilchen von Beispiel 1, mit einem Eisengehalt von 2 Mol/l, werden tropfenweise 2 ml einer 0,1 molaren Silbemitrat-Lösung unter Rühren bei 20°C gegeben. Die überschüssige Elektrolytlösung wird durch Dialyse mit einem 40kD-Filter mit dest. Wasser dialysiert, bis das Dialysat eine elektrische Leitfähigkeit von < 10 µS/cm besitzt. Die entstehende Dispersion ist sedimentations- und magnetfeldstabil und kann zur Herstellung eines parenteralen Therapeutikums bei bakteriellen Entzündungsprozessen im Körper Anwendung finden. Die Adsorption der superparamagnetischen Aggregate im Blutkreislauf kann mit Hilfe der Kernspin-Tomographie beobachtet werden.

### Beispiel 5:

20 ml der kleinen superparamagnetischen Eindomänenteilchen von Beispiel 2, mit einem Eisengehalt von 2 Mol/l, werden mit 2 ml einer radioaktiven Gallium-67-Citrat-Lösung mit einer Aktivität von 400 MBq (Mega Becquerel) und einer effektiven Dosis von 48 Sv (Sievert) versetzt. Die superparamagnetischen Eindomänenteilchen haben einen mittleren Teilchendurchmesser von ca. 14 nm. Die entstehende Dispersion ist sedimentations- und magnetfeldstabil und kann zur Herstellung eines parenteralen Radiopharmakons dienen, das zur Diagnose und Therapie von vulnerablen Plaques, sowie der Restenose nach Ballondilatation oder Stentimplantation eingesetzt werden kann. Durch den T1 und T2-Effekt der sehr kleinen superparamagnetischen Eindomänenteilchen ist eine Anreicherung der Teilchen in den Gefäßwänden mit Hilfe der Kernspin-Tomographie möglich.

Eine Diagnose und Therapie von Glioblastomen mit diesen radioaktiv citratbeschichteten kleinen superparamagnetischen Eindomänenteilchen ist ebenfalls möglich.

### Beispiel 6:

20 ml der kleinen superparamagnetischen Aggregate von Beispiel 2, mit einem Eisengehalt von 2 Mol/l, werden mit 2 ml einer radioaktiven Gallium-67-Citrat-Lösung mit einer Aktivität von 400 MBq und einer effektiven Dosis von 48 Sv versetzt. Die superparamagnetischen Aggregate haben einen mittleren Teilchendurchmesser von ca. 80 nm. Die entstehende Dispersion ist sedimentationsstabil und kann zur Herstellung eines parenteralen Radiopharmakons dienen. Die superparamagnetischen Aggregate von Beispiel 2 sind für Diagnose und Therapie von malignen Lebertumoren im Sinne der lokoregionären Radiotherapie (Radioembolisation) anwendbar.

### Beispiel 7:

20 ml der kleinen superparamagnetischen Eindomänenteilchen von Beispiel 2, mit einem Eisengehalt von 1 Mol/l, werden mit 4 ml einer 0,1 millimolaren Pentaethylenhexamin-Lösung versetzt. Zu dieser Dispersion werden dann radioaktiven Jodid-123-Lösung mit einer Aktivität von 300 MBq und einer effektiven Dosis von 2,3 Sv gegeben. Die superparamagnetischen Eindomänenteilchen haben einen mittleren Teilchendurchmesser von ca. 14 nm. Die entstehende Dispersion ist sedimentations- und magnetfeldstabil und kann zur Herstellung eines parenteralen Radiopharmakons dienen.

Für die Kopplung von gewebespezifischen Bindungssubstanzen, wie Antikörper von CD 30-Rezeptoren von Hodgkin-Lymphomen oder Antikörper von GD2- Rezeptoren von Neuroblastomen, werden die freien Amingruppen des Pentaethylenhexamins verwendet.

### Beispiel 8:

20 ml der kleinen superparamagnetischen Eindomänenteilchen von Beispiel 2, mit einem Eisengehalt von 2 Mol/l, werden mit 2 ml einer 0,1 molaren Platin II-chlorid-Lösung unter Rühren bei 20 °C tropfenweise versetzt. Die überschüssige Elektrolytlösung wird durch Dialyse mit einem 40 kD-Filter mit dest. Wasser dialysiert, bis das Dialysat eine elektrische Leitfähigkeit von < 10 µS/cm besitzt. Die superparamagnetischen Eindomänenteilchen haben einen mittleren Teilchendurchmesser von ca. 10 nm. Die entstehende Dispersion ist sedimentations- und magnetfeldstabil und kann zur Herstellung eines platinhaltigen Katalysators dienen.

### Beispiel 9:

20 ml der kleinen superparamagnetischen Eindomänenteilchen von Beispiel 2, mit einem Eisengehalt von 2 Mol/l, werden mit 1,5 ml einer Mischung von 1 ml 0,1 molaren Platin II-chlorid-Lösung und 0,5 ml 0,1 molaren Rhenium III-chlorid-Lösung unter Rühren bei 20°C tropfenweise versetzt. Die überschüssige Elektrolytlösung wird durch Dialyse mit einem 40 kD-Filter mit dest. Wasser dialysiert, bis das Dialysat eine elektrische Leitfähigkeit von < 10 µS/cm besitzt. Die superparamagnetischen Eindomänenteilchen haben einen mittleren Teilchendurchmesser von ca. 10 nm. Die entstehende Dispersion ist sedimentations- und magnetfeldstabil und kann zur Herstellung eines platin-rhenium-haltigen Katalysators dienen.

### Muster 10 (Beispiel 10):

Die superparamagnetischen Eindomänenteilchen von Beispiel 8 werden mit 1 molarer Oxalsäurelösung versetzt und unter Erwärmen auf 70°C die Eisenoxidanteile gelöst. In der gelben Lösung befinden sich die sehr kleinen nanometergroßen Platinteilchen. Die überschüssige Elektrolytlösung wird durch Dialyse mit einem 3 kD-Filter Filter mit dest. Wasser dialysiert, bis das Dialysat eine elektrische Leitfähigkeit von < 10 µS/cm besitzt. Die entstehende Dispersion von Platinteilchen ist sedimentations- und magnetfeldstabil und kann zur Herstellung eines platinhaltigen Katalysators dienen.

## Patentansprüche

1. Stabilisierte superparamagnetische Teilchen, bestehend aus superparamagnetischen Eindomänenteilchen aus Eisenhydroxid, Eisenoxidhydrat, Eisenoxid-, Eisenmischoxid- oder Eisen, die eine Teilchengröße im Bereich von 2 und 50 Nanometer haben, oder Aggregaten davon, die eine Teilchengröße im Bereich von10 bis 1000 Nanometer haben, oder Gemischen davon, jeweils stabilisiert auf ihrer Oberfläche durch aliphatische Di- oder Polycarbonsäuren oder Derivate davon, die eine Aggregation und Sedimentation im Schwerefeld verhindern, **dadurch gekennzeichnet, dass** die superparamagnetischen Eindomänenteilchen auf ihrer Oberfläche geladene Ionen chemischer Elemente gebunden tragen, wobei die Ionen bis zu einem Metallionengehalt von 5 Mol% des Eisengehaltes der Eindomänenteilchen gebunden sind und positiv geladene Metallionen sind, die aus der Gruppe ausgewählt sind, die aus Ionen der chemischen Elemente Kupfer, Silber, Gold, Eisen, Nickel, Kobalt, Gallium, Thallium, Bismut, Palladium, Rhenium, Rhodium, Ruthenium, Platin, Technetium, Indium, Iridium, Osmium, Radium, Selen, Vanadium, Yttrium, Zirkon, seltenen Erden, Gemischen davon und radioaktiven Isotopen dieser Elemente besteht.

2. Teilchen nach Anspruch 1, **dadurch gekennzeichnet, dass** die Metallionen aus der Gruppe der radioaktiven Isotope, bestehend aus ⁵²Fe, ⁶⁷Ga, ^{99m}Tc, ¹¹³In, ¹⁸⁸Rh, ¹⁹²Ir, ¹⁹⁸Au, ²⁰¹Tl und ²²³Ra ausgewählt sind.

3. Teilchen nach Anspruch 1, **dadurch gekennzeichnet, dass** die positiv geladenen Metallionen aus der Gruppe ausgewählt sind, die aus Metallionen der chemischen Elemente Kupfer, Silber, Gold, Platin, Palladium, Osmium, Rhenium, Rhodium, Ruthenium, Vanadium und Gemischen davon besteht.

4. Stabilisierte superparamagnetische Teilchen, bestehend aus superparamagnetischen Eindomänenteilchen aus Eisenhydroxid, Eisenoxidhydrat, Eisenoxid-, Eisenmischoxid- oder Eisen, die eine Teilchengröße im Bereich von 2 und 50 Nanometer haben, oder Aggregaten davon, die eine Teilchengröße im Bereich von10 bis 1000 Nanometer haben, oder Gemischen davon, jeweils stabilisiert auf ihrer Oberfläche durch aliphatische Di- oder Polycarbonsäuren oder Derivate davon, die eine Aggregation und Sedimentation im Schwerefeld verhindern, **dadurch gekennzeichnet, dass** die superparamagnetischen Eindomänenteilchen auf ihrer Oberfläche geladene Ionen chemischer Elemente gebunden tragen, wobei die geladenen Ionen Nichtmetallionen sind, die über eine Polyethylenimin-Brücke an die Oberfläche der superparamagnetischen Eindomänenteilchen gebunden sind.

5. Teilchen nach Anspruch 4, **dadurch gekennzeichnet, dass** die geladenen Ionen solche der radioaktiven Isotope ¹³N, ¹⁵O, ¹⁸F, ¹²³J oder Gemische davon sind.

6. Teilchen nach Anspruch 1 oder 4, **dadurch gekennzeichnet, dass** die superparamagnetischen Eindomänenteilchen auf ihrer Oberfläche stabilisiert sind durch Äpfelsäure, Weinsäure, Citronensäure, Asparaginsäure oder Gemische davon.

7. Teilchen nach Anspruch 1 oder 4, **dadurch gekennzeichnet, dass** die superparamagnetischen Eindomänenteilchen und die Teilchen der stabilen, abbaubaren Aggregate aus Eisenhydroxid, Eisenoxidhydrat, γ-Fe₂O₃, Fe₃O₄, aus den Eisenmischoxiden der allgemeinen Formel mMO.nFe₂O₃, worin M die zweiwertigen Metallionen Fe, Co, Ni, Mn, Be, Mg, Ca, Ba, Sr, Cu, Zn, Pt oder Gemische davon bedeuten, oder aus den Mischoxiden der allgemeinen Formel mFe₂O₃.nMe₂O₃, worin Me die dreiwertigen Metallionen Al, Cr, Bi, seltene Erdmetalle oder Gemische davon bedeuten, oder Eisen bestehen, wobei m und n ganze Zahlen im Bereich von 1 bis 6 sind.

8. Teilchen nach Anspruch 1 oder 4, **dadurch gekennzeichnet, dass** die superparamagnetischen Eindomänenteilchen auf ihrer Oberfläche zusätzlich zu den stabilisierenden Carbonsäuren und den positiv geladenen Ionen chemischer Elemente eine gewebespezifische Bindungssubstanz oder eine pharmakologisch wirksame Substanz oder ein Gemisch davon aufweisen.

9. Teilchen nach einem der Ansprüche 1 bis 8, **dadurch gekennzeichnet, dass** dieR₁-Relaxivität der superparamagnetischen Eindomänenteilchen im Bereich von 2 bis 50 liegt und das Verhältnis der Relaxivitäten R₂/R₁ kleiner als 5 ist.

10. Verfahren zur Herstellung von stabilisierten superparamagnetischen Teilchen nach Anspruch 1 aus durch Carbonsäuren stabilisierten Eindomänenteilchen oder deren Aggregaten, **dadurch gekennzeichnet, dass** die stabilisierten superparamagnetischen Eindomänenteilchen und Aggregate oder Gemische davon mit Lösungen vermischt werden, die positiv geladene Metallionen enthalten bei einer Konzentration der Lösungen im Bereich von 0,001 millimolar bis 1 molar und einem Verhältnis von Ionen chemischer Elemente zu Eisen von <10 Mol-% bei einer Temperatur von 5 bis 70 °C und anschließend die Teilchendispersion von überschüssigen Ionen gereinigt wird.

11. Verfahren zur Herstellung von stabilisierten superparamagnetischen Teilchen nach Anspruch 4, aus durch Carbonsäuren stabilisierten Eindomänenteilchen oder deren Aggregaten, **dadurch gekennzeichnet, dass** die stabilisierten superparamagnetischen Eindomänenteilchen und Aggregate oder Gemische davon mit Lösungen vermischt werden, die Nichtmetallionen enthalten bei einer Konzentration der Lösungen im Bereich von 0,001 millimolar bis 1 molar und einem Verhältnis von Ionen chemischer Elemente zu Eisen von <10 Mol-% bei einer Temperatur von 5 bis 70 °C und anschließend die Teilchendispersion von überschüssigen Ionen gereinigt wird, wobei die Lösungen mit den Nichtmetallionen vor dem Vermischen mit den superparamagnetischen Teilchen mit einem Polyethylenimin in Kontakt gebracht werden, oder die mit Polyethylenimin behandelten superparamagnetischen Teilchen mit den Lösungen, die Nichtmetallionen enthalten, in Kontakt gebracht werden.

12. Pharmakologisch wirksame Zubereitung, bestehend aus einem pharmakologisch annehmbaren Träger und superparamagnetischen Eindomänenteilchen oder Aggregaten nach Anspruch 1 oder 4.

13. Zubereitung nach Anspruch 12, **dadurch gekennzeichnet, dass** die Eindomänenteilchen der Aggregate zusätzlich zu der stabilisierenden Carbonsäure und den Metallionen eine gewebespezifische Bindungssubstanz oder eine pharmakologisch wirksame Substanz oder ein Gemisch davon angekoppelt an die stabilisierende(n) Carbonsäure(n) enthalten.

14. Verwendung der stabilisierten superparamagnetischen Teilchen nach Anspruch 1 oder 4 als magnetische Ionenaustauscher und magnetische Adsorbentien für Separationsverfahren, zur Herstellung von extrem kleinen Metallteilchen, als Magnetteitchen für die in vitro Diagnostik, gegebenenfalls unter Einwirkung von Magnetfeldern.

15. Verwendung der stabilisierten superparamagnetischen Teilchen nach Anspruch 1 oder 4 zur Herstellung eines Bakteriostaticums, zur Herstellung eines Radiopharmakons, zur Herstellung eines MR-Kontrastmittels, zur Herstellung eines Mittels zur Tumorschädigung, zur Verhinderung von Restenosen, zur Bekämpfung von Entzündungskrankheiten, zur Funktionskontrolle von Organen, zum magnetischen drug targeting.

## Claims

1. Stabilised superparamagnetic particles consisting of superparamagnetic single domain particles of iron hydroxide or iron oxihydrate or iron oxides or iron mixed oxide or iron having a particle size ranging between 2 and 50 nanometers, or aggregates thereof having a particle size ranging from 10 to 1000 nanometers, or mixtures thereof, respectively stabilised on their surface by means of aliphatic dicarbonic or polycarbonic acids or derivatives thereof, which prevent an aggregation and sedimentation in gravity, **characterised in that** the superparamagnetic single domain particles carry charged ions of chemical elements bound to their surface, wherein the ions are bound up to a metal ion portion of 5 % mol of the iron portion of the single domain particles and wherein the ions are positively charged metal ions selected from the group consisting of ions of the chemical elements copper, silver, gold, iron, nickel, cobalt, gallium, thallium, bismuth, palladium, rhenium, rhodium, ruthenium, platinum, technetium, indium, iridium, osmium, radium, selenium, vanadium, yttrium, zirconium, rare earths, mixtures thereof and radioactive isotopes of said elements.

2. Particles according to claim 1, **characterised in that** the metal ions are selected from the group of radioactive isotopes consisting of ⁵²Fe, ⁶⁷Ga, ^{99m}Tc, ¹¹³In, ¹⁸⁸Rh, ¹⁹²Ir, ¹⁹⁸Au, ²⁰¹Tl and ²²³Ra.

3. Particles according to claim 1, **characterised in that** the positively charged metal ions are selected from the group consisting of metal ions of the chemical elements copper, silver, gold, platinum, palladium, osmium, rhenium, rhodium, ruthenium, vanadium and mixtures thereof.

4. Stabilised superparamagnetic particles consisting of superparamagnetic single domain particles of iron hydroxide or iron oxihydrate or iron oxides or iron mixed oxide or iron having a particle size ranging between 2 and 50 nanometers, or aggregates thereof having a particle size ranging from 10 to 1000 nanometers, or mixtures thereof, respectively stabilised on their surface by means of aliphatic dicarbonic or polycarbonic acids or derivatives thereof, which prevent an aggregation and sedimentation in gravity, **characterised in that** the superparamagnetic single domain particles carry charged ions of chemical elements bound to their surface, wherein the charged ions are non-metal ions which are bound by means of a polyethylenimine bridge to the surface of the superparamagnetic single domain particles.

5. Particles according to claim 4, **characterised in that** the charged ions are those of the radioactive isotopes ¹³N, ¹⁵O, ¹⁸F, ¹²³J or mixtures thereof.

6. Particles according to claim 1 or 4, **characterised in that** the superparamagnetic single domain particles are stabilised on their surface by means of malic acid, tartaric acid, citric acid, aspartic acid or mixtures thereof.

7. Particles according to claim 1 or 4, **characterised in that** the superparamagnetic single domain particles and the particles of the stable and degradable aggregates consist of iron hydroxide, iron oxihydrate, γ-Fe₂O₃, Fe₃O₄, the iron mixed oxides of the general formula mMO·nFe₂O₃ wherein M refers to the bivalent metal ions Fe, Co, Ni, Mn, Be, Mg, Ca, Ba, Sr, Cu, Zn, Pt or mixtures thereof or of the mixed oxides of the general formula m Fe₂O_{3·}nMe₂O₃ wherein Me refers to the trivalent metal ions Al, Cr, Bi, rare earths or mixtures thereof or of iron wherein m and n are integers ranging from 1 to 6.

8. Particles according to claim 1 or 4, **characterised in that** the superparamagnetic single domain particles comprise on their surface in addition to the stabilising carbonic acids and the positively charged ions of chemical elements a tissue-specific binding substance or a pharmacologically active substance or a mixture thereof.

9. Particles according to any one of the claims 1 to 8, **characterised in that** the R₁-relaxivity of the superparamagnetic single domain particles lies in the range from 2 to 50 and the ratio of the relaxivities R₂/R₁ is less than 5.

10. Method for the manufacture of stabilised superparamagnetic particles according to claim 1 from carbonic acid-stabilised single domain particles or their aggregates, **characterised in that** the method comprises mixing the stabilised superparamagnetic single domain particles and aggregates or mixtures thereof with solutions containing positively charged metal ions wherein the concentration of the solutions lies in the range from 0.001 millimolar to 1 molar and wherein further the ratio of ions of chemical elements to iron is <10 mol-% and wherein the temperature is 5 to 70 °C and subsequently the particle dispersion is rid of excess ions.

11. Method for the manufacture of stabilised superparamagnetic particles according to claim 4 from carbonic acid-stabilised single domain particles or their aggregates, **characterised in that** the method comprises mixing the stabilised superparamagnetic single domain particles and aggregates or mixtures thereof with solutions containing non-metal ions wherein the concentration of the solutions lies in the range from 0.001 millimolar to 1 molar and wherein further the ratio of ions of chemical elements to iron is <10 mol-% and wherein the temperature is 5 to 70°C and subsequently the particle dispersion is rid of excess ions, wherein the solutions having the non-metal ions are brought into contact with a polyethylenimine before mixing with the superparamagnetic particles or the superparamagnetic particles treated with polyethylenimine are brought into contact with the solutions containing the non-metal ions.

12. Pharmacologically active preparation consisting of a pharmacologically acceptable carrier and superparamagnetic single domain particles or aggregates according to claim 1 or 4.

13. Preparation according to claim 12 **characterised in that** the single domain particles of the aggregates comprise coupled to the stabilising carbonic acid(s) in addition to the stabilising carbonic acid and the metal ions a tissue-specific binding substance or a pharmacologically active substance or a mixture thereof.

14. Use of the stabilised superparamagnetic particles according to claim 1 or 4 as magnetic ion exchangers and magnetic adsorbents for separation procedures, for the manufacture of extremely small metal particles, as magnetic particles for in vitro diagnosis, optionally under the action of magnetic fields.

15. Use of the stabilised superparamagnetic particles according to claim 1 or 4 for the manufacture of a bacteriostatic, for the manufacture of a radiopharmaceutical agent, for the manufacture of a MR contrast agent, for the manufacture of an agent for the purpose of tumour destruction, for the prevention of restenosis, for the combating of inflammatory diseases, for the control of organ functions, for the purpose of magnetic drug targeting.

## Revendications

1. Particules superparamagnétiques stabilisées, constituées de particules superparamagnétiques à un seul domaine d'hydroxyde de fer, d'hydrate d'oxyde de fer, d'oxyde de fer, d'oxyde mixte de fer ou de fer, qui ont une taille dans une gamme entre 2 et 50 nm, ou d'agrégats d'entre elles, qui ont une taille dans une gamme de 10 à 1000 nanomètres, ou de mélanges d'entre eux, respectivement stabilisées à leur surface par des acides di- ou polycarboxyliques aliphatiques ou des dérivés d'entre eux, qui empêchent une agrégation ou une sédimentation dans le champ de gravitation, **caractérisées en ce que** les particules superparamagnétiques à un seul domaine portent liés à leur surface des ions chargés d'éléments chimiques, moyennant quoi les ions sont liés jusqu'à une teneur en ions métalliques de 5 % en moles de la teneur en fer des particules à un seul domaine et sont des ions métalliques chargés positivement qui sont sélectionnés parmi le groupe constitué des ions des éléments chimiques cuivre, argent, or, fer, nickel, cobalt, gallium, thallium, bismuth, palladium, rhénium, rhodium, ruthénium, platine, technétium, indium, iridium, osmium, radium, sélénium, vanadium, yttrium, zirconium, terres rares, des mélanges d'entre eux et des isotopes radioactifs de ces éléments.

2. Particules selon la revendication 1, **caractérisées en ce que** les ions métalliques sont sélectionnés dans le groupe des isotopes radioactifs constitué de ⁵²Fe, ⁶⁷Ga, ^{99m}Tc, ¹¹³In, ¹⁸⁸Rh ¹⁹²Ir ¹⁹⁸Au, ²⁰¹Tl et ²²³Ra.

3. Particules selon la revendication 1, **caractérisées en ce que** les ions métalliques chargés positivement sont choisis parmi le groupe constitué des ions métalliques des éléments chimiques cuivre, argent, or, platine, palladium, osmium, rhénium, rhodium, ruthénium, vanadium et des mélanges d'entre eux.

4. Particules superparamagnétiques stabilisées, constituées de particules superparamagnétiques à un seul domaine d'hydroxyde de fer, d'hydrate d'oxyde de fer, d'oxyde de fer, d'oxyde mixte de fer ou de fer, qui ont une taille dans une gamme entre 2 et 50 nm, ou d'agrégats d'entre elles, qui ont une taille dans une gamme de 10 à 1000 nanomètres, ou de mélanges d'entre eux, respectivement stabilisées à leur surface par des acides di- ou polycarboxyliques aliphatiques ou des dérivés d'entre eux, qui empêchent une agrégation ou une sédimentation dans le champ de gravitation, **caractérisées en ce que** les particules superparamagnétiques à un seul domaine portent liés à leur surface des ions chargés d'éléments chimiques, moyennant quoi les ions chargés sont des ions non métalliques chargés, qui sont liés par un pont de polyéthylène-imine à la surface des particules superparamagnétiques à un seul domaine.

5. Particules selon la revendication 4, **caractérisées en ce que** les ions chargés sont des ions des isotopes radioactifs ¹³N, ¹⁵O, ¹⁸F, ¹²³I ou des mélanges d'entre eux.

6. Particules selon la revendication 1 ou 4, **caractérisées en ce que** les particules superparamagnétiques à un seul domaine sont stabilisées à leur surface par de l'acide malique, de l'acide tartrique, de l'acide citrique, de l'acide aspargique ou des mélanges d'entre eux.

7. Particules selon la revendication 1 ou 4, **caractérisées en ce que** les particules superparamagnétiques à un seul domaine et les particules des agrégats stables, dégradables sont constituées d'hydroxyde de fer, d'hydrate d'oxyde de fer, de γ-Fe₂O₃, Fe₃O₄, d'oxydes mixtes de fer de formule générale mMO·nFe₂O₃, dans laquelle M représente des ions métalliques bivalents Fe, Co, Ni, Mn, Be, Mg, Ca, Ba, Sr, Cu, Zn, Pt ou des mélanges d'entre eux, ou d'oxydes mixtes de formule générale mFe₂O₃·nMe₂O₃, dans laquelle Me représente des ions métalliques trivalents Al, Cr, Bi, des métaux de terre rare ou des mélanges d'entre eux, ou de fer, moyennant quoi m et n sont des nombres entiers de 1 à 6.

8. Particules selon la revendication 1 ou 4, **caractérisées en ce que** les particules superparamagnétiques à un seul domaine comportent sur leur surface, en plus des acides carboxyliques stabilisants et des ions chargés positivement d'éléments chimiques, une substance de liaison spécifique des tissus ou une substance pharmacologiquement active ou un mélange d'entre elles.

9. Particules selon l'une des revendications 1 à 8, **caractérisées en ce que** la relaxivité R₁ des particules superparamagnétiques à un seul domaine est dans une gamme de 2 à 50 et le rapport des relaxivités R₂/R₁ est inférieur à 5.

10. Procédé de fabrication de particules superparamagnétiques stabilisées selon la revendication 1 constituées de particules à un seul domaine stabilisées par des acides carboxyliques ou d'agrégats d'entre elles, **caractérisé en ce qu'**on mélange les particules superparamagnétiques à un seul domaine stabilisées et les agrégats ou les mélanges d'entre elles avec des solutions qui contiennent des ions métalliques chargés positivement à une concentration dans les solutions dans une gamme de 0,001 millimoles à 1 mole et à un rapport des ions d'éléments chimiques au fer < 10 % en moles, à une température de 5 à 70° C, et qu'ensuite on purifie la dispersion de particules des ions en excès.

11. Procédé de fabrication de particules superparamagnétiques stabilisées selon la revendication 4 constituées de particules à un seul domaine stabilisées par des acides carboxyliques ou d'agrégats d'entre elles, **caractérisé en ce qu'**on mélange les particules superparamagnétiques à un seul domaine stabilisées et les agrégats ou les mélanges d'entre elles avec des solutions qui contiennent des ions non métalliques à une concentration dans les solutions dans une gamme de 0,001 millimoles à 1 mole et à un rapport des ions d'éléments chimiques au fer < 10 % en moles, à une température de 5 à 70° C, et qu'ensuite on purifie la dispersion de particules des ions en excès, moyennant quoi on met en contact, avant le mélange avec les particules superparamagnétiques, les solutions contenant les ions non métalliques avec un polyéthylène-imine ou on met en contact les particules superparamagnétiques traitées avec du polyéthylène-imine avec les solutions qui contiennent des ions non métalliques.

12. Préparation pharmacologiquement active, constituée d'un support pharmacologiquement acceptable et de particules superparamagnétiques à un seul domaine ou d'agrégats selon la revendication 1 ou 4.

13. Préparation selon la revendication 12, **caractérisée en ce que** les particules à un seul domaine des agrégats contiennent, en plus de l'acide carboxylique stabilisant et les ions métalliques une substance de liaison spécifique des tissus ou une substance pharmacologiquement active ou un mélange d'entre elles couplée(s) à(aux) (l')acide(s) carboxylique(s) stabilisant(s).

14. Utilisation des particules superparamagnétiques stabilisées selon la revendication 1 ou 4 comme échangeur d'ions magnétique et adsorbant magnétique pour des procédés de séparation, pour fabriquer des particules magnétiques extrêmement petites, à titre de particules magnétiques pour le diagnostic in vitro, éventuellement sous l'effet de champs magnétiques.

15. Utilisation des particules superparamagnétiques stabilisées selon la revendication 1 ou 4 pour préparer un agent bactériostatique, pour préparer un produit radiopharmaceutique, pour préparer une substance de contraste de RM, pour préparer un agent anti-tumoral, pour prévenir les resténoses, pour lutter contre les maladies inflammatoires, pour le contrôle de fonction d'organes, pour le ciblage magnétique du médicament.
